# EUROPÄISCHE PATENTSCHRIFT

(11) **EP 2 086 497 B1**
(45) Veröffentlichungstag und Bekanntmachung des Hinweises auf die Patenterteilung: **17.07.2013**
(21) Anmeldenummer: 07729196.1
(22) Anmeldetag: 16.05.2007
(51) Int. Cl.: A61K 8/92, A61K 8/02, A61K 8/365, A61K 8/44, A61Q 15/00

(54) **KOSMETISCHE ODER DERMATOLOGISCHE FORMULIERUNG MIT MANDELSÄURE UND HILFSSTOFFE**
COSMETIC OR DERMATOLOGICAL FORMULATION WITH MANDELIC ACID AND AUXILIARIES
FORMULATION COSMÉTIQUE OU DERMATOLOGIQUE COMPRENANT DE L'ACIDE MANDÉLIQUE ET DES ADJUVANTS

(30) Priorität: 26.10.2006 EP 06122976
(43) Veröffentlichungstag der Anmeldung: 12.08.2009
(73) Patentinhaber: Beiersdorf AG, 20253 Hamburg (DE)
(72) Erfinder: BIEL, Stefan, 21077 Hamburg (DE); TERSTEGEN, Lara, 20253 Hamburg (DE); KUX, Ulrich, 22559 Hamburg (DE); SCHULZ, Ulrike, 06484 Quedlinburg (DE); RIPKE, Sabine, 22527 Hamburg (DE)
(86) Internationale Anmeldenummer: PCT/EP2007/054748
(87) Internationale Veröffentlichungsnummer: WO 2008/049650

(56) Entgegenhaltungen:
- EP-A- 0 571 677
- EP-A- 0 775 486
- EP-A- 1 190 640
- EP-A- 1 787 629
- WO-A-03/053388
- WO-A-2005/102256
- WO-A-2005/105026

## Beschreibung

Kosmetische oder dermatolgische Formulierung mit Mandelsäure und Hilfsstoffe

Die Erfindung betrifft eine kosmetische oder dermatologische Formulierung umfassend Mandelsäure, Antitranspirantien und Hilfsstoffe. Die Formulierung weist eine reduzierte Klebrigkeit gegenüber bekannten Formulierungen auf.

Vor allem aus ästhetischen Gründen werden transparente und transluzente kosmetische Produkte von vielen Verbrauchern bevorzugt. Transparente Formulierungen kommen so z. B. häufig als Deo oder Antitranspirant (AT) zum Einsatz. Diese lassen sich heutzutage durch folgende Technologien realisieren:
1. wässrig-alkoholische Formulierungen
2. Wasser-in-Silikon-Emulsionen
3. Mikro-Emulsionen

Die wässrig alkoholischen Deo- und AT-Formulierungen basieren zumeist auf Wasser und Alkohol als Medium, Deo- und Antitranspirantmittel als Wirkstoffe sowie Parfüm, Löslichkeitsvermittler und Verdicker (zumeist auf Kohlenhydratbasis) als zusätzliche Agenzien. Sie werden vom Verbraucher als frisch und kühlend empfunden, sind aber gleichzeitig mit einer ganzen Reihe an Nachteilen behaftet. So ist beispielsweise die Applikation vor allem auf frisch rasierter Haut durch den Alkoholgehalt mit Unverträglichkeiten verbunden. Ein weiterer großer Nachteil ist die Tatsache, dass in derartige Systeme keine größeren Ölmengen eingearbeitet werden können. Durch den für eine hocheffektive Wirkleistung erforderlichen hohen Gehalt an Antitranspirantsalz verbleibt nach der Applikation auf der Haut ein weißer Rückstand, der vom Verbraucher als überaus störend empfunden wird. Durch die technologisch bedingte Abwesenheit einer ausreichend großen Ölphase kann dieser allerdings nicht kaschiert werden. Darüber hinaus führt die Verwendung von Kohlenhydrat-Verdickern zu einer hohen Klebrigkeit des Produktes nach dem Verdunsten des Alkohols.

In der WO 2005/105026 werden transparente kosmetische und/oder dermatologische Formulierung offenbart, die mindestens einen Antitranspirant-Wirkstoff und/oder Deodorant-Wirkstoff, mindestens eine α-Hydroxycarbonsäure und Wasser umfassen. Als Hydroxysäure ist Mandelsäure bevorzugt zu wählen.

Bei Verwendung von Mandelsäure als α-Hydroxysäure, wie in der WO 2005/105026 beschrieben, kann der Eigengeruch der Mandelsäure zu Geruchsproblemen führen.

Zur Überdeckung kann man Parfume zusetzen. Dies führt aber häufig zu Instabilitäten und Einbussen hinsichtlich optischer Attraktivität, Transparenz des Kosmetikums.

Wünschenswert ist es, das man insbesondere in Deo/AT-Produkte, die aus olfaktorischen Gründen Parfume enthalten, diese stabil in kosmetische oder dermatologische Zubereitungen umfassend Mandelsäure einarbeiten kann.

Insbesondere ist es wünschenswert eine kosmetische und/oder dermatologische Formulierungen bereit zu stellen, die bevorzugt transparent ist und sich durch eine minimierte Klebrigkeit auszeichnet. Insbesondere bestand die Aufgabe darin eine Deo- oder Antitranspirantformulierung bereit zu stellen, die transparent ist und keinerlei Eintrübung aufweist, die sich durch eine minimierte Klebrigkeit auszeichnet und die eine definierte Fließgrenze zur optimierten Ausbringung und Applikation besitzt.

Gelöst wird das Bündel an Aufgaben durch eine kosmetische Formulierung entsprechend Anspruch 1. Gegenstand der Unteransprüche sind vorteilhafte Ausführungsformen der erfindungsgemäßen Zubereitung. Des weiteren umfasst die Erfindung die Verwendung derartiger Zubereitungen.

Die erfindungsgemäße Formulierung lässt sich zu einer viskosen bis pastösen Formulierung gelieren und ermöglicht die Bereitstellung einer transparenten und wenig klebrigen kosmetischen Zubereitung, insbesondere eine Antitranspirant- bzw. Deodorantzubereitung.

Der Vorteil ist, dass EDTA Metallionen komplexiert und dadurch Probleme, die bei der Geruchsentstehung durch den Metallionen-katalysierter Abbau von Mandelsäure zu Benzaledehyd entstehen können, behoben werden können. Des weiteren hilft EDTA Verfärbungen zu vermeiden.

Di- und Triglyceride mit einem Ethoxylierungsgrad von ca. 5 bis 100, insbesondere PEG-40 hydrogenated castor oil, dienen als Lösungsvermittler für Parfümöle.

Damit ist es möglich Parfümöle transparent in das System einzubinden.

Der Einsatz eines Hilfsstoffes gewählt aus der Gruppe Ethylendinitrilotetraessigsäure (EDTA) und/oder ein Salz davon und/oder Calcium-dinatrium-ethylendiamintetraacetat ist somit prädestiniert.

Insbesondere die Kombination aus EDTA und PEG-40 hydrogenated castor oil ist sinnvoll, um ein Produkt mit optimalen visuellen (Transparenz und Farbe) und olfaktorischen Eigenschaften (kein Eigengeruch, dafür Einarbeitung von Parfumölen in idealen Mengen) zu erhalten.

Weiterer erfindungsgemäßer Vorteil ist, dass die bei der Vergelung von Mandelsäure mit ACH entstehenden Einzelkomplexe innherhalb des Gelnetzwerks in ihrer Größe durch die Zugabe von EDTA und/oder PEG-40 hydrogenated castor oil so eingestellt werden können, dass das gewünschte rheologische Profil (Gel mit Fließgrenze, s. unten)) optimal eingestellt und erreicht wird.

Ein zusätzlicher Benefit ist die positive Beeinflussung der Parfumfreisetzung aus der erfindungsgemäßen Zubereitung mit einem idealen Freisetzungsprofil über den Tag, der durch die Hilfsstoffe kombiniert mit der erwähnten Maskierung von Benzaldehydgeruch durch das EDTA.

Insbesondere ist es erfindungsgemäß möglich stabil und transparent Parfume in die Zubereitung einzuarbeiten.

Durch die überraschend einfache Kombination von Antitranspirantwirkstoffen, Mandelsäure und mindestens einem Hilfsstoff in Wasser lassen sich kosmetische und dermatologische Formulierungen herstellen, die keinerlei objektiv als auch subjektiv empfundene Klebrigkeit aufweisen, bevorzugt transparent sind und Parfum enthalten können.

Die Hydroxyphenylessigsäure oder auch Phenylglykolsäure mit der Formel H₅C₆ -CH(OH) -COOH, C₈H₈O₃ ist bekannt unter dem Namen Mandelsäure. Die Mandelsäure ist gut löslich in Wasser, Alkohol, Ether u. 2-Propanol. Synthetisch erhält man die (±)-Mandelsäure aus Benzaldehyd und Blausäure über das α-Hydroxynitril (Cyanohydrin) und dessen saure Hydrolyse entsprechend Abbildung 1:

### Abbildung 1: Herstellung Mandelsäure

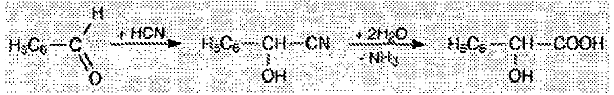

Mittels der Mandelsäure, läßt sich überraschenderweise eine AT- bzw. Deodorantzubereitung aber auch eine beliebige kosmetische Zubereitung herstellen, die die vorteilhaften Eigenschaften, wie Transparenz, geringe Klebrigkeit, Parfumbestandteile und darüber hinaus auch die Einstellung einer bestimmten Fließgrenze der Zubereitung ermöglicht. Des weiteren zieht die erfindungsgemäße Formulierung sehr schnell ohne Rückstände zu hinterlassen in die Haut ein.

Darüberhinaus trägt Mandelsäure durch seine keratinolytischen Eigenschaften zur Regeneartion der Achselhaut bei und wirkt im sauren Millieu der Achsel bakteriostatisch. Aufgrund dieser Eigenschaften ist Mandelsäure hervorragend auch als aktive Komponente in kosmetischen und insbesondere Deo/AT-Produkten geeignet.

Die Fließgrenze oder Fließpunkt ist eine Bezeichnung für die kleinste Schubspannung, oberhalb derer ein plastischer Stoff sich rheologisch wie eine Flüssigkeit verhält (DIN 1342-1: 1983-10). Die Bestimmung der Fließgrenze erfolgt durch Aufnahme einer Fließkurve (DIN 53019: 1980-05; DIN 53214: 1982-02). Der erhaltene Wert hängt stark von der Zeitskala (Belastungsrate) ab, die der Messung zugrunde liegt. Dies ist unabhängig davon, ob die Messung mit einem schubspannungs- oder drehzahlgesteuerten Viskosimeter erfolgt. Kurze Zeitskalen (schnelle Belastungen) ergeben in der Regel höhere Werte für die Fließgrenze. Eine zu hohe Fließgrenze kann Ursache von Verlaufstörungen sein. Andererseits lässt sich mit geeignet bemessener Fließgrenze die Neigung der flüssigen Formulierung zum Ablaufen unterdrücken.

Die erfindungsgemäße Zubereitung liegt daher vorteilhaft als Gel- bzw. Hydrogel vor und weist ein Fließgrenze auf, wodurch die Ausbringung und Applikation gegenüber den Zubereitungen aus dem Stand der Technik verbessert ist.

Die erfindungsgemäße Kombination aus AT-Wirkstoff, Mandelsäure, Hilfsstoff und Wasser ermöglicht über einen einzigartigen Verdickungsmechanismus die Herstelltung einer insbesondere transparenten kosmetischen Zubereitung. Der Anwender hat somit erstmalig eine wasserklare und dennoch überaus wirksame Zubereitung zur Hand. Die erfindungsgemäße Zubereitung ist in Gelform bequem zu applizieren und weist eine angenehmes Hautgefühl aufgrund der fehlenden Klebrigkeit auf.

Als Antitranspirantwirkstoff lassen sich vorteilhaft saure Aluminium- und/oder Aluminium/Zirkoniumsalze in wässriger Lösung einarbeiten. Hierbei beziehen sich die beschriebenen Konzentrationsbereiche auf die so genannten Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Die nachfolgende Auflistung vorteilhaft einzusetzender Antitranspirant-Wirker soll in keiner Weise einschränkend sein:

Aluminium-Salze (der empirischen Summenformel [Al₂(OH)ₘClₙ], wobei m+n=6):
- Aluminiumchlorhydrat [Al₂(OH)₅Cl] x H₂O
   Standard Al-Komplexe: Locron L, Locron LIC, Locron LIF (Clariant), Chlorhydrol (Reheis), ACH-303 (Summit), Aloxicoll L (Giulini).
   Aktivierte Al-Komplexe: Reach 501 (Reheis), Aloxicoll 51 L
- Aluminiumsesquichlorhydrat [Al₂(OH)_{4,5}Cl_{1,5}] x H₂O
   Standard Al-Komplexe: Aloxicoll 31 L (Giulini), Westchlor 186 (Westwood Chemicals)
   Aktivierte Al-Komplexe: Reach 301 (Reheis)
- Aluminiumdichlorhydrat [Al₂(OH)₄Cl₂] x H₂O

Aluminium-Zirkonium-Salze:
- Aluminium/Zirkonium Trichlorhydrex Glycin [Al₄Zr(OH)₁₃Cl_{3]} x H₂O x Gly Standard Al/Zr-Komplexe: Rezal 33GC (Reheis), AZG-7164 (Summit)
- Aluminium/Zirkonium Tetrachlorhydrex Glycin [Al₄Zr(OH)₁₂Cl₄] x H₂O x Gly
   Standard Al/Zr-Komplexe: Rezal 36, Rezal 36G, Rezal 36 GC (Reheis), AZG-368 (Summit), Zirkonal L435G (Giulini), Westchlor ZR 35 BX5, Westchlor ZR 41 (Westwood Chemicals)
- Aluminium/Zirkonium Pentachlorhydrex Glycin [Al₈Zr(OH)₂₃Cl₅] x H₂O x Gly
   Standard Al/Zr-Komplexe: Rezal 67 (Reheis), Zirkonal L540, Zirkonal L530 PG (Giulini), Westchlor ZR 80B (Westwood Chemicals)
- Aluminium/Zirkonium Octachlorhydrex Glycin [Al₈Zr(OH)₂₀Cl₈] x H₂O x Gly: Westchlor ZR 82B

Ebenso vorteilhaft können aber auch Glycin-freie Aluminium/Zirkonium-Salze eingesetzt werden.

Die Antitranspirant-Wirkstoffe werden in den erfindungsgemäßen Formulierungen in einer Menge von 1 bis 35 Gew.-%, vorzugsweise von 1 bis 20 Gew.-%, eingesetzt.

Vorteilhaft können erfindungsgemäßen Zubereitungen auch Desodorantien zugesetzt werden. Den üblichen kosmetischen Desodorantien liegen unterschiedliche Wirkprinzipien zugrunde. Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.

Alle für Desodorantien gängigen Wirkstoffe können vorteilhaft genutzt werden, beispielsweise Geruchsüberdecker wie die gängigen Parfume oder deren Parfümbestandteile, Geruchsabsorber, beispielsweise die in der DE 40 09 347 beschriebenen Schichtsilikate, von diesen insbesondere Montmorillonit, Kaolinit, Illt, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Bevorzugt umfasst die erfindungsgemäße Zubereitung ein oder mehrere Parfume und/oder deren Bestandteile.

Die Menge der Desodorantien (eine oder mehrere Verbindungen) in den Zubereitungen beträgt vorzugsweise 0,01 bis 10 Gew.-%, bevorzugt 0,05 bis 5 Gew.-% bezogen auf das Gesamtgewicht der Zubereitung.

Mittels der Mandelsäure und den AT-wirkstoff - Aluminium-Salz - läßt sich überraschenderweise ein Hydrogel herstellen, dass die geforderten Eigenschaften, wie Transparenz und geringe Klebrigkeit aufweist. Darüber hinaus zieht die erfindungsgemäße Formulierung sehr schnell ohne Rückstände zu hinterlassen in die Haut ein. Tabelle 1 zeigt den Vergleich verschiedener transparenter Fomulierungen in einem Sensorik-Research-Panel, bestehend aus 8 geschulten Prüfern. Dazu wurden die Proben in definierter Menge auf die Haut aufgetragen und anhand einer Bewertungsskala bewertet (1 = nicht klebrig; 10 = stark klebrig).

**Tabelle 1**

| | erfindungsgemäßes Beispiel | Vergleichsbeispiele | | |
|---|---|---|---|---|
| | Transparentes Hydrogel | Nanoemulsio n | Wasser-in-Silikon-Emulsion | Wässrig-alkoholische Formulierung |
| Einzugsvermög en in Sekunden | 95 | 179 | 153 | 106 |
| Klebrigkeit Skala von 1-10 | 3,4 | 5,2 | 6,5 | 5,3 |

Als besonders vorteilhaft hat sich eine Kombination aus Mandelsäure und Aluminium Clorohydrat gezeigt, wobei das Verhältnis Aluminum Chlorohydrat zu Mandelsäure 15:1 bis 1:1, bevorzugt 12:1 bis 2:1, insbesondere 10:1 bis 2,5:1.

Der oder die weitere Hilfsstoffe werden gewählt aus der Gruppe polyethoxyllierter Fettsäuren von Mono-, Di- und Triglyceriden mit einem Ethoxylierungsgrad von ca. 5 bis 100, insbesondere von ca. 20 bis 80. Bevorzugt sind polyethoxylierte hydrogenierte castor Öle, insbesondere PEG-40 hydrogenated castor oil. PEG-40 hydrogenated castor oil ist als Handelprodukt unter dem Namen Solutor, Eumulgin oder Fancol erhältlich.

Als bevorzugte Beispiele seien PEG-40 hydrogenated castor oil und PEG-60 hydrogenated castor oil erwähnt. Die polyethoxyllierter Fettsäuren von Mono-, Di- und Triglyceriden, insbesondere PEG-40 hydrogenated castor oil, lassen sich vorteilhaft in Kombination mit Parfum einsetzen.

Parfum ist für viele Kosmetika ein essentieller Bestandteil, aber er führt häufig zu Problemen und Instabilitäten.

Mit dem oder den Hilfsstoffen, insbesondere dem Hilfsstoff oder Lösevermittler Solutor (PEG-40), lassen sich Parfume in das Mandelsäure Gel einarbeiten ohne das es trüb oder instabil wird. D.h. es ist weiterhin eine vorteilhafte Transparenz und Stabilität von Mandelsäure Gelen, wie sie in der WO 2005105026 beschrieben sind, gegeben.

Gebräuchliche Anteile an Hilfsstoffen liegen vorteilhaft im Bereich von 1 bis 10 Gew.-%. insbesondere etwa 4 bis 7 Gew.%, bevorzugt im Bereich von 5 bis 6 Gew.-%, jeweils bezogen auf die Gesamtmasse der Zubereitung.

Als Hilfsstoffe können daher Lösevermittler, insbesondere PEG-40 HYDROGENATED CASTOR OIL (Warenbez.: Solutor, Eumulgin, Fancol, Cremophor), dies insbesondere in Kombination mit Parfum und/oder deren Bestandteilen, gewählt werden.

Als Hilfsstoff wird EDTA, Ethylendinitrilotetraessigsäure, und/oder ein Salz davon und/oder Calcium-dinatrium-ethylendiamintetraacetat eingesetzt Bevorzugt ist auch die Kombination zweier Hilfsstoffe, z. B. EDTA und PEG-40.

Die erfindungsgemäßen kosmetischen und dermatologischen Zubereitungen können kosmetische Zusatzstoffe enthalten, wie sie üblicherweise in solchen Zubereitungen verwendet werden, z.B. Konservierungsmittel, Bakterizide, UV-Filter, Antioxidantien, wasserlösliche Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Die Herstellung der erfindungsgemäßen gelförmigen Zubereitung erfolgt vorteilhaft durch Lösen der Mandelsäure in Wasser. Anschließend erfolgt die Zugabe der wässrigen AT-Wirkstoffe, insbesondere Aluminum-Salz-Lösung, sowie die Hilfsstoffe und/oder Zusatzstoffe unter Rühren.

Zur Applikation der Zubereitung lassen sich herkömmliche Packmittel für Deodorantien und/oder Antitransipirantien verwenden, z. B. Stiftdispenser, Geldispenser, Tuben und Roller.

Die Angaben der Beispiele beziehen sich auf Gewichtsprozent bezogen auf die Gesamtmasse der Zubereitung.

**Beispiele**

| | 1. Stick Gew.-% | 2. Roller Gew.-% | 3. Stick * Gew.-% |
|---|---|---|---|
| Wasser | 70,8 | 71,9 | 71,8 |
| Trisodium EDTA | 1,0 | 1,0 | - |
| PEG-40 Hydrogenated Castor Oil | 5,0 | 5,0 | 5,0 |
| Aluminum Chlorohydrate (50% Lsg.) | 20,0 | 20,0 | 20,0 |
| Parfum | 1,0 | 1,0 | 1,0 |
| Mandelsäure | 2,2 | 1,1 | 2,2 |

Als PEG-40 Hydrogenated Castor Oil kann beispielsweise Cremophor RH 410 von BASH (Ludwigshafen, Deutschland) eingesetzt werden.

Aluminiumchlorohaydrat wird beispielsweise als 50 % Lösung in Wasser unter dem Handelsnamen CHLORHYDROL 50 % Solution von Reheis Inc. (Berkeley Heights, NJ, USA) angebotent.

## Patentansprüche

1. Kosmetische und/oder dermatologische Formulierung umfassend mindestens einen Antitranspirant-Wirkstoff, Mandelsäure und Wasser, **dadurch gekennzeichnet, dass** die Formulierung mindestens einen Hilfsstoff, gewählt aus der Gruppe Ethylendinitrilotetraessigsäure (EDTA) und/oder ein Salz davon und/oder Calciumdinatrium-ethylendiamintetraacetat, umfasst,
ausgenommen die Formulierungen I bis IV:
Formulierung I:
20 Gew.-% Aluminium Chlorohydrat
0,1 Gew.-% Disodium EDTA
1 Gew.-% Parfüm
2,2 Gew.-% Mandelsäure
5,0 Gew. Gew-% PEG-40 hydrogenated Castor Oil
71,7 Gew.-% Wasser
Formulierung II: eine klare Gelformulierung mit Gasblasen darin, umfassend die folgenden Komponenten
Komponente A: 2.2 g Mandelsäure in 70,8 Wasser (für 15 Minuten mit einem Magnetrührer bei Raumtemperatur gerührt)
Komponente B: 1.0 g of Parfüm in 5.0 g PEG 40 hydrogenated castor oil Komponente C: 20.0 g of wäßriger ACH Lösung (20 Gew.-%)
Komponente D: wäßrige trisodium EDTA Lösung (20 Gew. %)
hergestellt mittels der folgenden Prozedur
1. Gib Komponente A zu Komponente B und rühre für 20 Minuten bei Raumtemperatur (200 min-1; paddle stirrer RW 20 DZM.n, 72 W, IP 20/KL O von IKA Labortechnik, Bern, Schweiz) bis eine klare Lösung gebildet wird.
2. Gib Komponente C zur Lösung und rühre bei Raumtemperatur für 45 Minuten.
3. Gib Komponente D zu und rühre bei Raumtemperatur für 15 Minuten.
Die resultierende Mischung wird mit einem der folgenden Geräte behandelt zur Einarbeitung von Gasblasen.
(a) "Zauberstab" von ESGE, Deutschland (Model SG 2000, 200 watts, KB 5 min.): 5 sec bei Einstellung 1 und 10 min bei Einstellung 2;
(b) Ultraturrax (IKA T 50 basic, 100 watts, IP 21): 2 Minuten bei 5,200 min-1;
(c) Homocenta (Model S100 LS90, 2 PS) 2810 min-1; Mischung wird zweimal durch das Gerät gegeben;
(d) Die Mischung wird auch mit Druckluft behandelt: 30 sec bei 3 L/min und 1.5 min bei 2-5 Umin.;
Formulierung III: eine klare Gelformulierung mit Beads darin umfassend die folgenden Komponenten
Komponente A: 2.2 g Mandelsäure in 70,5 Wasser (für 20 Minuten bei 25 °C gerührt).
Komponente B: 1.0 g of Parfüm in 5.0 g PEG 40 hydrogenated castor oil (Rühren für 20 Minuten bei 25 °C)
Komponente C: 20.0 g of wäßriger ACH Lösung (20 Gew.-%)
Komponente D: wäßrige trisodium EDTA Lösung (20 Gew. %).
Komponente E: 0,3 g Beads
hergestellt mittels der folgenden Prozedur
1. Gib Komponente B zu Komponente A und rühre für 20 Minuten bei 25 °C (0,5 m/s Becomix-Mischer 15 CD von der A.Berents GmbH & Co KG, Stuhr, Deutschland)
2. Gib Komponente C hinzu und rühre bei 25 °C für 60 Minuten.
3. Gib Komponente D zu und rühre bei 25 °C für 10 Minuten.
4. Gib Komponente E hinzu und rühre bei 25 °C für 10 Minuten.
Als Beads (Komponente E) werden die folgenden kommerziell erhältlichen Produkte verwendet:
(1) Cosmospheres GMM-S (von Pelletech Ltd., Schweiz; Durchmesser 1.1-1.5 mm; grün; umfassend Mannitol, microcrystalline Cellulose, CI 77289 (Chromhydroxid Grün) und Milchsäure);
(2) Cosmospheres BMM-M (von Pelletech Ltd., Schweiz; Durchmesser 1.1-1.5 mm; grün; umfassend Mannitol, microcrystalline Cellulose, CI 77289 (Pigment Blue 15) und Milchsäure);
(3) Beads Cosmo YS-S (von Pelletech Ltd., Schweiz; Durchmesser 0,5-1,0 mm; gelb; umfassend Lactose, microcrystalline Cellulose, Helianthus annuus, CI 77492);
(4) Unispheres RP-5 72 (von Induchem AG, Schweiz; Durchmesser 0.5-0.9 mm; pink; umfassend Lactose, Cellulose, Hydroxypropylmethylcellulose, Panthenyl triacetate, CI 73360); Unispheres UE-507 (von Indchem AG, Schweiz; Durchmesser 0.5-0.9 mm; purpur; umfassend Lactose, Cellulose, Hydroxypropylmethylcellulose, Tocopherylacetat, CI 77007 (Pigment Blue 29);
(5) Cosmospheres Beads BCG2-L (von Pelletech Ltd., Schweiz; Durchmesser 1.5-2.0 mm; blau, glitzernd; umfassend Lactose, Polyethylenterephthalate, microcrystalline Cellulose, Acrylates Copolymer und Pigment Blue 15) und Cosmospheres Beads WCG-G-C-L (von Pelletech Ltd., Schweiz; Durchmesser 1.5-2.0 mm; golden, glitzernd; umfassend Lactose, Polyethyleneterephthalate, microcrystalline Cellulose, Schellack, Acrylates Copolymer, mica, Titandioxid und Eisenoxid);
und
Formulierung IV: eine klare Gelzubereitung mit Gasblasen darin umfassend die folgenden Komponenten
Komponente A: 2.2 kg Mandelsäure in 70.8 kg Wasser (gerührt bei 25°C für 45 Minuten bei 18 min-1 und für 2 Minuten bei 2320 min-1; Krieger MMU-1 00 der Krieger AG, Muttenz, Schweiz)
Komponente B: 1.0 kg Parfum in 5.0 kg PEG 40 hydrogenated castor oil (gerührt für 2 Minuten bei 25°C und 18 min-1; Krieger MMU-1 00)
Komponente C: 20.0 g wäßrige ACH Lösung (20 Gew.-%)
Komponente D: wäßrige trisodium EDTA Lösung (20 Gew.-%)
hergestellt mittels der folgenden Prozedur
1. Gib Komponente B zu Komponente A und rühre für 24 Minuten bei 25°C (bei 18 min-1; Krieger MMU-100)
2. Gib Komponente C hinzu und rühre bei 25°C für 60 Minuten.
3. Gib Komponente D hinzu und rühre bei 25°C für 10 Minuten.
4. Homogenisiere bei 25°C für 8 Minuten bei 2320 min-1 (Krieger MMU-100).
5. Bei 25°C für 2 Stunden stehen lassen.
6. Bearbeite mit einem Hansa Mixer (Hansa Industrie-Mixer GmbH & Co KG, Stuhr, Deutschland) unter den folgenden Bedingungen
Mischkopf 600 min-1
Pumpe 120 Uh
Luftdruck 6,9 bar
Gasgehalt 0,4 NUmin
Systemdruck 0,7 bar
Temperatur 24°C
Überschuß (theoretisch) 15%
Überschuß (aktuell) 8,19%

2. Formulierung nach Anspruch 1 umfassend ein oder mehrere Parfüme und/oder deren Bestandteile.

3. Formulierung nach einem der vorstehenden Ansprüche, **dadurch gekennzeichnet, dass** der Antitranspirant-Wirkstoff aus der Gruppe der Aluminium-Salze, bevorzugt Aluminium-Chlorohydrat oder Aluminium-Zirkonium-Salze, gewählt wird.

4. Formulierung nach einem der voranstehenden Ansprüche, **dadurch gekennzeichnet, dass** die Mandelsäure, in einer Menge von 0,1 bis 10 Gew.%, vorzugsweise von 0,1 bis 8 Gew.% bezogen auf die Gesamtmasse der Formulierung, eingesetzt wird.

5. Formulierung nach einem der voranstehenden Ansprüche, **dadurch gekennzeichnet, dass** der oder die Hilfsstoffe in einer Menge von 1 bis 10 Gew.%, vorzugsweise von 4 bis 7 Gew.%, bezogen auf die Gesamtmasse der Formulierung, eingesetzt werden.

6. Verwendung einer kosmetischen Formulierung nach mindestens einem der vorhergehenden Ansprüche zur Auftragung auf die menschliche Haut.

7. Verwendung einer Formulierung nach mindestens einem der vorhergehenden Ansprüche zur Herstellung eines transparenten Kosmetikums, insbesondere eines Deodorant- und/oder Antitranspirant-Hydrogels.

## Claims

1. Cosmetic and/or dermatological formulation comprising at least one antiperspirant active ingredient, mandelic acid and water, **characterized in that** the formulation comprises at least one auxiliary selected from the group ethylenedinitrilotetraacetic acid (EDTA) and/or a salt thereof and/or calcium/disodium ethylenediaminetetraacetate, with the exception of formulations I to IV:
Formulation I:
20% by weight aluminium chlorohydrate
0.1% by weight disodium EDTA
1% by weight perfume
2.2% by weight mandelic acid
5.0% by weight PEG-40 hydrogenated castor oil
71.7% by weight water
Formulation II: a clear gel formulation with gas bubbles therein, comprising the following components
Component A: 2.2 g mandelic acid in 70.8 g of water (stirred for 15 minutes with a magnetic stirrer at room temperature)
Component B: 1.0 g of perfume in 5.0 g of PEG 40 hydrogenated castor oil
Component C: 20.0 g of aqueous ACH solution (20% by weight)
Component D: aqueous trisodium EDTA solution (20% by weight)
prepared by means of the following procedure
1. Add component A to component B and stir for 20 minutes at room temperature (200 min⁻¹; paddle stirrer RW 20 DZM.n, 72 W, IP 20/KL O from IKA Labortechnik, Bern, Switzerland) until a clear solution is formed.
2. Add component C to the solution and stir at room temperature for 45 minutes.
3. Add component D and stir at room temperature for 15 minutes.
The resulting mixture is treated with one of the following instruments to incorporate gas bubbles.
(a) "Zauberstab" from ESGE, Germany (model SG 2000, 200 watts, KB 5 min) : 5 sec at setting 1 and 10 min at setting 2;
(b) Ultraturrax (IKA T 50 basic, 100 watts, IP 21): 2 minutes at 5.200 min⁻¹;
(c) Homocenta (model S100 LS90, 2 PS) 2810 min⁻¹; mixture is passed through the instrument twice;
(d) the mixture is also treated with compressed air: 30 sec at 3 l/min and 1.5 min at 2-5 l/min;
Formulation III: a clear gel formulation with beads therein comprising the following components
Component A: 2.2 g of mandelic acid in 70.5 g of water (stirred for 20 minutes at 25°C).
Component B: 1.0 g of perfume in 5.0 g of PEG 40 hydrogenated castor oil (stirring for 20 minutes at 25°C)
Component C: 20.0 g of aqueous ACH solution (20% by weight)
Component D: aqueous trisodium EDTA solution (20% by weight)
Component E: 0.3 g of beads
prepared by means of the following procedure
1. Add component B to component A and stir for 20 minutes at 25°C (0.5 m/s Becomix mixer 15 CD from A. Berents GmbH & Co KG, Stuhr, Germany)
2. Add component C and stir at 25°C for 60 minutes.
3. Add component D and stir at 25°C for 10 minutes.
4. Add component E and stir at 25°C for 10 minutes.
The beads (component E) used are the following commercially available products:
(1) Cosmospheres GMM-S (from Pelletech Ltd., Switzerland; diameter 1.1-1.5 mm; green; comprising mannitol, microcrystalline cellulose, CI 77289 (Chromium Hydroxide Green) and lactic acid);
(2) Cosmospheres BMM-M (from Pelletech Ltd., Switzerland; diameter 1.1-1.5 mm; green; comprising mannitol, microcrystalline cellulose, CI 77289 (Pigment Blue 15) and lactic acid);
(3) Beads Cosmos YS-S (from Pelletech Ltd., Switzerland; diameter 0.5-1.0 mm; yellow; comprising lactose, microcrystalline cellulose, Helianthus annuus, CI 77492);
(4) Unispheres RP-5 72 (from Induchem AG, Switzerland; diameter 0.5-0.9 mm; pink; comprising lactose, cellulose, hydroxypropylmethylcellulose, Panthenyl triacetate, CI 73360); Unispheres UE-507 (from Induchem AG, Switzerland; diameter 0.5-0.9 mm; purple; comprising lactose, cellulose, hydroxypropylmethylcellulose, tocopheryl acetate, CI 77007 (Pigment Blue 29);
(5) Cosmospheres Beads BCG2-L (from Pelletech Ltd., Switzerland; diameter 1.5-2.0 mm; blue, glittering; comprising lactose, polyethylene terephthalate, microcrystalline cellulose, Acrylates Copolymer and Pigment Blue 15) and Cosmospheres Beads WCG-G-C-L (from Pelletech Ltd., Switzerland, diameter 1.5-2.0 mm; golden, glittering; comprising lactose, polyethylene terephthalate, microcrystalline cellulose, shellac, Acrylates Copolymer, mica, titanium dioxide and iron oxide);
and
Formulation IV: a clear gel preparation with gas bubbles therein comprising the following components
Component A: 2.2 kg of mandelic acid in 70.8 kg of water (stirred at 25°C for 45 minutes at 18 min⁻¹ and for 2 minutes at 2320 min⁻¹; Krieger MMU-100 from Krieger AG, Muttenz, Switzerland)
Component B: 1.0 kg of perfume in 5.0 kg of PEG 40 hydrogenated castor oil (stirred for 2 minutes at 25°C and 18 min⁻¹; Krieger MMU-100)
Component C: 20.0 g of aqueous ACH solution (20% by weight)
Component D: aqueous trisodium EDTA solution (20% by weight)
prepared by the following procedure
1. Add component B to component A and stir for 24 minutes at 25°C (at 18 min⁻¹; Krieger MMU-100)
2. Add component C and stir at 25°C for 60 minutes.
3. Add component D and stir at 25°C for 10 minutes.
4. Homogenize at 25°C for 8 minutes at 2320 min⁻¹ (Krieger MMU-100) .
5. Leave to stand at 25°C for 2 hours.
6. Process with a Hansa Mixer (Hansa Industrie-Mixer GmbH & Co KG, Stuhr, Germany) under the following conditions
Mixing head 600 min⁻¹
Pump 120 l/h
Air pressure 6.9 bar
Gas content 0.4 l(stp)/min
System pressure 0.7 bar
Temperature 24°C
Excess (theoretical) 15%
Excess (actual) 8.19%

2. Formulation according to Claim 1, comprising one or more perfumes and/or constituents thereof.

3. Formulation according to one of the preceding claims, **characterized in that** the antiperspirant active ingredient is selected from the group of the aluminium salts, preferably aluminium chlorohydrate or aluminium-zirconium salts.

4. Formulation according to one of the preceding claims, **characterized in that** the mandelic acid is used in an amount of from 0.1 to 10% by weight, preferably from 0.1 to 8% by weight, based on the total mass of the formulation.

5. Formulation according to one of the preceding claims, **characterized in that** the auxiliary or auxiliaries are used in an amount of from 1 to 10% by weight, preferably from 4 to 7% by weight, based on the total mass of the formulation.

6. Use of a cosmetic formulation according to at least one of the preceding claims for application to the human skin.

7. Use of a formulation according to at least one of the preceding claims for producing a transparent cosmetic, in particular a deodorant and/or antiperspirant hydrogel.

## Revendications

1. Composition cosmétique et/ou dermatologique, comprenant au moins une substance active antisudorale, de l'acide mandélique et de l'eau, **caractérisée en ce que** la composition comprend au moins un adjuvant choisi dans le groupe constitué par l'acide éthylènedinitrilotétraacétique (EDTA) et/ou un sel de celui-ci et/ou de l'éthylènediaminetétraacétate de calcium et de disodium,
à l'exclusion des compositions I à IV :
Composition I :
20 % en poids d'aluminium chlorohydrate
0,1 % en poids de disodium EDTA
1 % en poids de parfum
2,2 % en poids d'acide mandélique
5,0 % en poids de PEG-40 hydrogenated castor oil
71,7 % en poids d'eau
Composition II : une formule de gel limpide contenant des bulles de gaz, comprenant les composants suivants
Composant A : 2,2 g d'acide mandélique dans 70,8 g d'eau (agité pendant 15 minutes à la température ambiante à l'aide d'un agitateur magnétique)
Composant B : 1,0 g de parfum dans 5,0 g de PEG 40 hydrogenated castor oil
Composant C : 20,0 q de solution aqueuse d'ACH (à 20 % en poids)
Composant D : solution aqueuse de trisodium EDTA (à 20 % % en poids)
préparée par la procédure suivante
1. Addition du composant A au composant B et agitation pendant 20 minutes à la température ambiante (200 min⁻¹ ; paddle stirrer RW 20 DZM.n, 72 W, IP 20/KL O d'IKA Labortechnik, Berne, Suisse) jusqu'à formation d'une solution limpide.
2. Addition du composant C à la solution et agitation à la température ambiante pendant 45 minutes.
3. Addition du composant D et agitation à la température ambiante pendant 15 minutes.
On traite le mélange résultant à l'aide d'un des appareils suivants pour l'incorporation de bulles de gaz.
(a) « Zauberstab » d'ESGE, Allemagne (modèle SG 2000, 200 watts, KB 5 min.) : 5 s au réglage 1 et 10 min. au réglage 2 ;
(b) Ultraturrax (IKA T 50 basic, 100 watts, IP 21) : 2 minutes à 5 200 min.⁻¹ ;
(c) Homocenta (modèle S100 LS90, 2 PS) 2 810 min.⁻¹ ; on fait passer deux fois le mélange dans l'appareil ;
(d) On traite également le mélange par de l'air comprimé : 30 s à 3 l/min et 1,5 min. à 2-5 l/min. ;
Composition III : une formule de gel limpide contenant des perles, comprenant les composants suivants
Composant A : 2,2 g d'acide mandélique dans 70,5 g d'eau (agitation pendant 20 minutes à 25 °C).
Composant B : 1,0 g de parfum dans 5,0 g de PEG 40 hydrogenated castor oil (agitation pendant 20 minutes à 25 °C)
Composant C : 20,0 g de solution aqueuse d'ACH (à 20 % en poids)
Composant D : solution aqueuse de trisodium EDTA (à 20 % en poids).
Composant E : 0,3 g de perles
préparée par la procédure suivante
1. Addition du composant B au composant A et agitation pendant 20 minutes à 25 °C (0,5 m/s mélangeur Becomix 15 CD de la Société A. Berents GmbH & Co KG, Stuhr, Allemagne)
2. Addition du composant C à ce mélange et agitation à 25 °C pendant 60 minutes.
3. Addition du composant D à ce mélange et agitation à 25 °C pendant 10 minutes.
4. Addition du composant E à ce mélange et agitation à 25 °C pendant 10 minutes.
En tant que perles (composant E) on utilise les produits suivants disponibles dans le commerce :
(1) Cosmospheres GMM-S (de Pelletech Ltd., Suisse ; diamètre 1,1-1,5 mm ; verstes ; comprenant mannitol, microcrystalline cellulose, CI 77289 (vert hydroxyde de chrome) et acide lactique) ;
(2) Cosmospheres BMM-M (de Pelletech Ltd., Suisse ; diamètre 1,1-1,5 mm ; vertes ; comprenant mannitol, microcrystalline cellulose, Cl 77289 (Pigment Blue 15) et acide lactique) ;
(3) Perles Cosmo YS-S (de Pelletech Ltd., Suisse ; diamètre 0,5-1,0 mm : jaunes ; comprenant lactose, microcrystalline cellulose, Helianthus annuus, CI 77492);
(4) Unispheres RP-5 72 (d'Induchem AG, Suisse ; diamètre 0,5-0,9 mm; roses ; comprenant lactose, cellulose, hydroxypropylmethylcellulose, panthenyl triacetate, CI 73360); Unispheres UE-507 (d'Induchem AG, Suisse ; diamètre 0,5-0,9 mm ; pourpres ; comprenant lactose, cellulose, hydroxypropylmethylcellulose, tocopheryl acétate, CI 77007 (Pigment Blue 29) ;
(5) Cosmospheres Beads BCG2-L (de Pelletech Ltd., Suisse ; diamètre 1,5-2,0 mm ; bleues, scintillantes ; comprenant lactose, polyethylene terephthalate, microcrystalline cellulose, acrylates copolymer et Pigment Blue 15) et Cosmospheres Beads WCG-G-C-L (de Pelletech Ltd., Suisse ; diamètre 1,5-2,0 mm ; dorées, scintillantes ; comprenant lactose, polyethylene terephthalate, microcrystalline cellulose, gomme-laque, acrylates copolymer, mica, dioxyde de titane et oxyde de fer) ;
et
Composition IV : une préparation en gel limpide contenant des bulles de gaz, comprenant les composants suivants
Composant A : 2,2 kg d'acide mandélique dans 70,8 kg d'eau (agitation à 25 °C pendant 45 minutes à 18 min.⁻¹ et pendant 2 minutes à 2 320 min.⁻¹ ; Krieger MMU-100 de la Société Krieger AG, Muttenz, Suisse)
Composant B : 1,0 kg de parfum dans 5,0 kg de PEG 40 hydrogenated castor oil (agitation pendant 2 minutes à 25 °C et 18 min.⁻¹ ; Krieger MMU-100)
Composant C : 20,0 g de solution aqueuse d'ACH (à 20 % en poids)
Composant D : solution aqueuse de trisodium EDTA (à 20 % en poids) préparée par la procédure suivante
1. Addition du composant B au composant A et agitation pendant 24 minutes à 25 °C (à 18 min.⁻¹ ; Krieger MMU-100)
2. Addition du composant C à ce mélange et agitation à 25 °C pendant 60 minutes.
3. Addition du composant D à ce mélange et agitation à 25 °C pendant 10 minutes.
4. fiomogénéisation à 25 °C pendant 8 minutes à 2 320 min.⁻¹ (Krieger MMU-100).
5. Abandon pendant 2 heures à 25 °C.
6. Traitement par un mélangeur Hansa (Hansa Industrie-Mixer GmbH & Co KG, Stuhr, Allemagne) dans les conditions suivantes
Tête de mélange 600 min.⁻¹
Pompe 120 l/h
Pression d'air 6,9 bars
Teneur en gaz 0,4 1 normal/min
Pression du système 0,7 bar
Température 24 °C
Excédent (théorique) 15 %
Excédent (réel) 8,19 %.

2. Composition selon la revendication 1, comprenant un ou plusieurs parfums et/ou leurs composants.

3. Composition selon l'une quelconque des revendications précédentes, **caractérisée en ce que** la substance active antisudorale est choisie dans le groupe des sels d'aluminium, de préférence aluminium chlorohydrate ou sels d'aluminium-zirconium.

4. Composition selon l'une quelconque des revendications précédentes, **caractérisée en ce qu'**on utilise l'acide mandélique en une quantité de 0,1 à 10 % en poids, de préférence de 0,1 à 8 % en poids, par rapport à la masse totale de la composition.

5. Composition selon l'une quelconque des revendications précédentes, **caractérisée en ce qu'**on utilise l'adjuvant ou les adjuvants en une quantité de 1 à 10 % en poids, de préférence de 4 à 7 % en poids, par rapport à la masse totale de la composition.

6. Utilisation d'une composition cosmétique selon au moins l'une quelconque des revendications précédentes, pour l'application sur la peau humaine.

7. Utilisation d'une composition cosmétique selon au moins l'une quelconque des revendications précédentes, pour la fabrication d'un produit cosmétique transparent, en particulier d'un hydrogel déodorant et/ou antisudoral.
